# EUROPEAN PATENT APPLICATION

(11) **EP 4 417 240 A1**
(43) Date of publication of application: **21.08.2024**
(21) Application number: 21960726.4
(22) Date of filing: 15.10.2021
(51) Int. Cl.: A61M 21/02, A61B 5/00, H04R 1/10

(54) **DEVICE FOR INDUCING SLEEP AND METHOD FOR OPERATING SAME**

(71) Applicant: LG Electronics Inc., Yeongdeungpo-gu Seoul 07336 (KR)
(72) Inventor: KIM, Hyungjin, Seoul 06772 (KR); CHO, Jasoo, Seoul 06772 (KR); NOH, Seungpyo, Seoul 06772 (KR); LEE, Jinsool, Seoul 06772 (KR)
(74) Representative: Vossius & Partner Patentanwälte Rechtsanwälte mbB
(86) International application number: PCT/KR2021/014412
(87) International publication number: WO 2023/063459

(57) **Abstract**

A device for inducing sleep according to the present invention comprises: a sensor configured to be wearable on a user's body, and contacting a part of the user's body to obtain a sleep-related signal; an output module that outputs a synthesized sound source obtained by mixing a first sound source for inducing the user's sleep and a second sound source different from the first sound source; and a control unit. The control unit transmits the sleep-related signal to an external device, receives sleep state information of the user according to an analysis of the sleep-related signal from the external device, and adjusts an output ratio of the first and second sound sources constituting the synthesized sound source and outputs the first and second sound sources, on the basis of the received sleep state information of the user. Accordingly, the user can fall asleep more comfortably, sleep soundly, and wake up refreshed.

## Description

### Technical Field

The present disclosure relates to a device for inducing sleep and a method of operating the same, and more particularly, to a device for inducing sleep, the device being configured to be wearable on a body of a user and capable of outputting a sound source for inducing sleep of the user, and a method of operating the device.

### Background Art

Recently, as there are a growing number of people having difficulty in sleeping, a need for sound sleep is increasing. Accordingly, a market for sleeponomics for satisfying a need for high-quality sleep is also growing. Particularly, there is a rapid growth in a market for sleep tech devices such as devices configured to provide various sleep-inducing content, autonomous sensory meridian responses (ASMRs), meditation music, etc., brain wave measurement headsets, etc. as a method of helping to have a sound sleep by stabilizing a brain activity using sound.

Meanwhile, types of observed brain waves vary depending on brain activities, and the brain waves are closely related to human sleep. For example, delta waves (0.5 to 4 Hz) mainly appear during a drowsy or sleeping state, and beta waves (14 to 30 Hz) are mainly observed during a wakefulness state. In addition, in a relaxed state, alpha waves (7 to 14 Hz) or theta waves (4 to 8 Hz) are mainly observed.

Patterns of brain waves observed during sleeping is largely classified into rapid eye movement (REM) sleep and non-REM sleep, and the non-REM sleep is generally subdivided into four stages according to a depth of sleep. Generally, the sleep tech devices help a user to have a sound sleep by stimulating brain waves to induce the user into a deep sleep stage when a light sleep stage is observed.

However, it is very difficult to continuously measure the brain waves without interfering with a sleep activity. In detail, a sensor needs to be in contact with a body of a user in which brain waves are easily measured to accurately measure the brain waves. However, a problem such that a sleep activity of the user may be disturbed due to wearing discomfort is present. In addition, there is such a problem that reliability of brain wave data being measured is worsened as a contact point for brain wave measurement becomes unstable due to movement of the user during a sleeping activity or a part of a device in contact with a body of the user is contaminated.

### Disclosure of Invention

### Technical Problem

Therefore, to obviate those problems, an aspect of the detailed description is to provide a device for inducing sleep and a method of operating the same.

In addition, an aspect of the present disclosure is to provide a device for inducing sleep, the device being configured to allow accurate and stable brain wave measurement even while a user is sleeping for a long period of time and minimize wearing discomfort, and a method of operating the device.

In addition, another aspect of the present disclosure is to provide a device for inducing sleep, the device being implemented such that an element in contact with a person's body to measure brain waves may be cleaned and detachably coupled to allow hygienic and stable brain wave measurement, and a method of operating the device.

In addition, another aspect of the present disclosure is to provide a device for inducing sleep, the device being configured to provide a personalized and customized sound source for sleeping, reduce sleep initiation time, and induce deep sleep as well as seamless wakefulness, and a method of operating the device.

### Solution to Problem

To achieve these and other advantages and in accordance with the purpose of the present disclosure, as embodied and broadly described herein, there is provided a device for inducing sleep, the device including: a main body configured to be wearable on a body of a user; a communication module configured to communicate with an external device; a sensor detachably coupled to the main body and configured to obtain a sleep-related signal by being in contact with a part of the body of the user; an output module configured to output a synthesized sound source obtained by mixing a first sound source for inducing sleep of the user with a second sound source other than the first sound source; and a control unit configured to control the communication module to transmit the obtained sleep-related signal to the external device and receive, from the external device, sleep state information of the user according to analysis of the sleep-related signal, wherein the control unit controls to output the synthesized sound source by adjusting an output ratio of the first sound source and the second sound source both constituting the synthesized sound source based on the received sleep state information of the user.

In an embodiment, in response to determining entry into a sleep initiation state based on the sleep state information of the user, the control unit may output the synthetic sound source by increasing the output ratio of the first sound source to the second sound source to a preset rate.

In an embodiment, while the output ratio of the first sound source to the second sound source is gradually increased, the control unit may control the output module to gradually decrease volume of the second sound source to be turned off.

In an embodiment, the control unit may continuously communicate with the external device to monitor a sleep state corresponding to a sleep-related signal obtained after the entry into the sleep initiation state and adjust an increase rate of the output ratio of the first sound source differently based on updated sleep state information.

In an embodiment, when the second sound source is selected through the external device, the control unit may transmit information related to a user interface configured to determine an initial volume value of the selected second sound source to the external device.

In an embodiment, the output ratio of the first sound source may be determined differently based on a type of the selected second sound source and the initial volume value of the second sound source, the initial volume value being determined using the user interface.

In an embodiment, in response to a sleep stage of the user reaching a preset level or above based on the sleep state information of the user, the control unit may output a synthesized sound source such that an output of the second sound source is turned off and an output of the first sound source is gradually reduced for a certain period of time to be turned off.

In an embodiment, while a rapid eye movement (REM) sleep state of the user is monitored based on the sleep state information of the user, the control unit may control the output module to output a sound source for inducing improvement of a memory, instead of the synthesized sound source, and when the REM sleep state is complete, to provide a synthesized sound source adjusted based on the sleep state information of the user.

In an embodiment, in response to a sleep stage of the user reaching a preset level or above based on the sleep state information of the user, the control unit may maintain the output module in an inactivated state, and when information about an alarm time is received from the external device, determine a time point when the output module is to be switched to an activated state based on the received information about the alarm time.

In an embodiment, the control unit may switch the output module to the activated state at the time point determined based on the received information about the alarm time, and the output module may output a sound source for inducing wake-up of the user.

In an embodiment, the control unit may collect information regarding a degree of adjustment of the output ratio of the first sound source to the second sound source during a preset period of time, and perform learning using the external device to output a synthesized sound source updated based on the collected information.

In an embodiment, the sensor may be configured to include a wiring structure to be detachably coupled to the main body, and include a plurality of electrodes disposed to be apart from each other.

In an embodiment, at least one of the plurality of electrodes may be disposed to be in contact with an inner ear of the user and made of a conductive silicone material.

Additionally, in accordance with an embodiment of the present disclosure, there is also provided a method of operating a device for inducing sleep wherein the device is configured to be wearable on a body of a user and communicate with an external device, the method including: obtaining a sleep-related signal, the obtaining being performed by a sensor configured to be in contact with a part of the body of the user; outputting a synthesized sound source obtained by mixing a first sound source for inducing sleep of the user with a second sound source other than the first sound source; transmitting the sleep-related signal obtained by the sensor to the external device, and receiving sleep state information of the user according to analysis of the sleep-related signal received from the external device; and outputting a synthesized sound source updated by adjusting an output ratio of the first sound source to the second sound source based on the received sleep state information of the user.

Additionally, in accordance with an embodiment of the present disclosure, there is also provided a computer program stored on a computer-readable recording medium for performing the method of claim 14 on a computer operated by one or more processors.

### Advantageous Effects of Invention

In accordance with the detailed description, a device for inducing sleep and a method of operating the same may be implemented such that a component in contact with a body of a user to measure brain waves is capable of being cleaned and detachably coupled to allow hygienic and stable brain wave measurement. In addition, a conductive silicon material may be used in consideration of wearing comfort to allow accurate and stable brain wave measurement even during sleeping for a long period of time and minimize wearing discomfort.

In addition, not only a sound source for inducing sleep, but also a synthesized sound source obtained by uniformly mixing preferred sound familiar to the user with a sound source for inducing sleep may be properly adjusted and provided according to a sleep state and a sleep stage so that the user may initiate sleep comfortably, have sound sleep, and wake up refreshed.

In addition, a personalized and customized sleep sound source may be provided, sleep initiation time may be reduced, deep sleep may be induced, and in a case of preparing wake-up, seamless wakefulness may be induced by stimulating brain waves to enable wakefulness step by step.

### Brief Description of Drawings

FIG. 1 is a block diagram for explaining respective elements of a device for inducing sleep according to the present disclosure and an external device in communication with the device for inducing sleep.
FIGS. 2A, 2B, and 2C illustrate the device for inducing sleep according to the present disclosure viewed from different directions. FIGS. 3A, 3B, 3C, 3D, and 3E are drawings for explaining a detailed configuration of at least some elements disassembled from the device for inducing sleep according to the present disclosure.
FIG. 4 is a flowchart for explaining a method of operating the device for inducing sleep according to the present disclosure.
FIG. 5A is a sleep graph showing an example of a sleep state and a sleep stage each corresponding to a biomedical signal of a user detected through the device for inducing sleep according to the present disclosure. FIG. 5B is a synthesized sound source output graph showing an example of adjusting a synthesized sound source according to the sleep state and the sleep stage of the user.
FIG. 6 illustrates an example of a user interface for controlling a synthesized sound source provided to an external device that communicates with the device for inducing sleep according to the present disclosure.
FIG. 7 illustrates an example of a user sleep analysis screen provided to the external device that communicates with the device for inducing sleep according to the present disclosure.
FIGS. 8A, 8B, 9A, and 9B are drawings for explaining examples of other structures of the device for inducing sleep according to the present disclosure.
FIG. 10 is a flowchart for explaining a service providing method for inducing, by a device for inducing sleep according to the present disclosure, sleep and wake-up in interoperation with a preset application.

### Mode for the Invention

Hereinafter, embodiments disclosed herein will be described in detail with reference to the accompanying drawings, and the same or similar elements are designated with the same numeral references, regardless of the numerals in the drawings, and their redundant description will be omitted. In general, a suffix such as "module" and "unit" may be used to refer to elements or components. Use of such a suffix herein is merely intended to facilitate description of the specification, and the suffix itself is not intended to give any special meaning or function. In addition, in describing the present disclosure, if a detailed explanation for a related known function or construction is considered to unnecessarily divert the gist of the present disclosure, such explanation has been omitted but would be understood by those skilled in the art. The accompanying drawings are used to help easily understand the technical idea of the present disclosure and it should be understood that the idea of the present disclosure is not limited by the accompanying drawings. The idea of the present disclosure should be construed to extend to any alterations, equivalents and substitutes besides the accompanying drawings.

Hereinafter, a device for inducing sleep according to the present disclosure and a method of operating the same are described in detail. The device for inducing sleep according to the present disclosure is configured to be wearable on a part of a user's body and include a sensor having a structure to be capable of being coupled or disassembled.

The 'device for inducing sleep' disclosed in this specification is configured to have a structure wearable on a part of a user's body and may be connected to at least one external device and perform wireless communication (or' data communication).

A 'sleep-related signal' disclosed in this specification includes both a signal corresponding to a user's movement and a biomedical signal of the user each detected through the sensor or a sensing unit in contact with a part of the user's body. In addition, the 'sleep-related signal' may further include additional information such as environmental information of a sleep place, e.g., a temperature, humidity, a level of illumination, a noise level, etc.

A 'first sound source' disclosed in this specification is a background sound source for inducing sleep, and may include, for example, a combination of one or more selected from binaural beats for adjusting brain waves by presenting sound of different frequency bands to both ears, white noise, and an autonomous sensory meridian response (ASMR) sound of rain. However, the first sound source is not limited thereto, and may include other background sound sources which are known or being developed for helping sleep, for example, a sound source at a tempo of 80 beat per minutes (BPM) or lower, personalized binaural beats (IAF-4Hz), proven classical music, fixed popular songs, etc.

A 'second sound source' disclosed in this specification is a customized sound source preferred by a user, and may include, for example, sound sources such as jazz music, popular songs, foreign pop songs which are frequently listened to, a list of recently popular sound sources, sound sources recommended based on a user's taste on a network, etc. A 'third sound source' disclosed in this specification may refer to a sound source for inducing improvement of a memory of the user. A 'fourth sound source' may refer to a sound source for inducing the user to wake up.

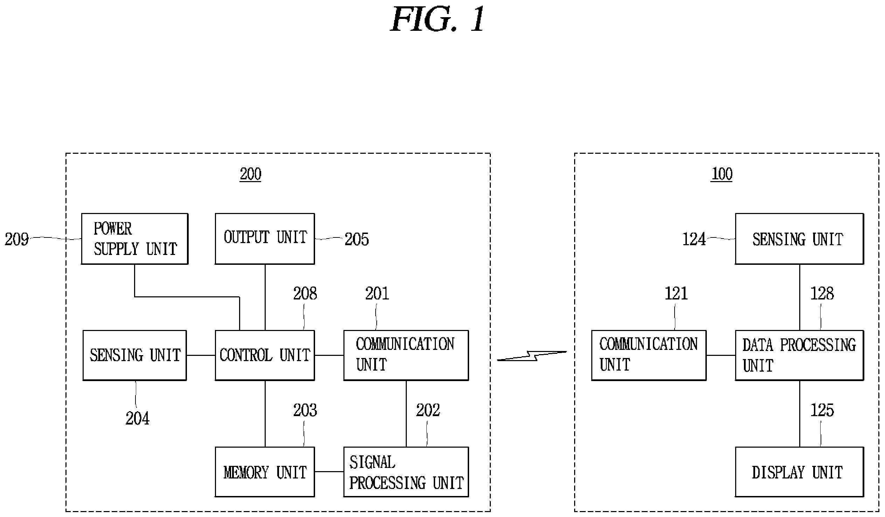

FIG. 1 is a block diagram for explaining respective elements of a device 200 for inducing sleep according to the present disclosure and an external device 100 in communication with the device 100 for inducing sleep. The device 200 for inducing sleep and the external device 100 each shown in FIG. 1 may include only a part of the elements shown in the drawing or further include other elements.

The device 200 for inducing sleep may include a communication unit 201, a signal processing unit 202, a memory unit 203, a sensing unit 204, an output unit 205, a power supply unit 209, and a control unit 208. The communication unit 215 or a communication module may communicate with the external device 100 using a wireless communication network such as a wireless fidelity (WiFi) network, a light fidelity (LiFi) network, etc. or a short-range communication network such as Bluetooth, radio frequency identification (RFID), infrared data association (IrDA), ultra-wideband (UWB), ZigBee, etc.

The signal processing unit 202 may preprocess (e.g., remove noise, correct offset, calculate root-mean-square (RMS)/power spectral density (PSD), etc.) and process signals obtained by the sensing unit 204 to perform analysis, and provide the preprocessed or processed signals to the communication unit 215 and/or the memory unit 203.

The sensing unit 204 may include a plurality of sensors configured to obtain sleep-related signals. In detail, the sensing unit 204 may be detachably coupled to a main body of the device 200 for inducing sleep and include one or more sensors configured to obtain a sleep-related signal by being in contact with a part of the user's body. In addition, the sensing unit 204 may further include a touch sensor, a pressure sensor, etc. each configured to recognize an input related to an operation of the device 200 for inducing sleep.

The one or more sensors may include, for example, a sensor configured to obtain a biomedical signal such as an electroencephalography (EEG) sensor, a photoplethysmogram (PPG) sensor, an electrodermal activity (EDA) sensor, an electrocardiogram (ECG) sensor, an electromyography (EMG) sensor, etc., a sensor configured to obtain a signal related to user movement, e.g., an acceleration sensor, a gyro sensor, a motion sensor, etc., and/or a sensor configured to obtain a signal related to an environment of a sleeping space, e.g., an illumination sensor, a temperature sensor, a humidity sensor, a noise measurement sensor, etc. In one embodiment, the sensor configured to obtain a biomedical signal (e.g., a brainwave signal) of a user may include a plurality of electrode sensors (at least two electrode sensors). For example, when the sensor configured to obtain a biomedical signal of a user includes three electrode sensors, the electrode sensors may be a measurement electrode (SIG) configured to measure an EEG signal, a reference electrode (REF), and a ground electrode (GND), respectively. In one embodiment, at least one of the plurality of electrode sensors may be disposed to be in contact with an inner ear of the user.

Additionally, in one embodiment, some of the plurality of electrode sensors, e.g., the measurement electrode (SIG) and the reference electrode (REF), may be arranged to be apart from each other to obtain great signal strength (voltage (RMS)). For example, the measurement electrode (SIG) may be placed to be in contact with the inner ear, and the reference electrode (REF) may be placed to be in contact with back of an ear of the user. More desirably, to further ensure a separation distance, the measurement electrode (SIG) may be implemented to be in contact with an inner ear of a left (right) ear, and the reference electrode (REF) and the ground electrode (GND) may be implemented to be in contact with an inner ear of a right (left) ear.

The memory unit 203 may store the sleep-related signal obtained through the sensing unit 204, e.g., an EEG signal, a movement signal, etc. When storing the sleep-related signal, the memory unit 203 may also store metadata such as reception time of the corresponding sleep-related signal, a recent sleep state (or a sleep stage), etc. Additionally, the memory unit 203 may store the first sound source and the second sound source according to the present disclosure, and/or a synthesized sound source obtained by mixing the first and second sound sources, a preferred sound source, a recommended background sound source, and relevant information. Additionally, the memory unit 203 may store a sleep pattern of the user (e.g., time needed for sleep initiation, a time point of entry into rapid eye movement (REM) sleep, etc.) and an output pattern of the synthesized sound source. Additionally, the memory unit 203 may store notification time and information related thereto.

The memory 203 may include one or more types of storage mediums including a flash memory type, a hard disk type, a multimedia card micro type, a card-type memory (e.g., secure digital (SD) or extreme digital (XD) memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk.

The output unit 205 or an output module outputs a synthesized sound source obtained by mixing a first sound source for inducing the user's sleep with a second sound source other than the first sound source. To do so, the output unit 205 may include a receiver, a speaker, a buzzer, etc. In one embodiment, when the device 200 for inducing sleep according to the present disclosure is equipped on both ears of the user, respective output units 205 of respective devices 200 for inducing sleep may output a synthesized sound source having different frequency bands. In another embodiment, when the device 200 for inducing sleep according to the present disclosure is implemented as a glasses-type device which is in contact with both eyes of a user, the output unit 205 may include a display capable of outputting visual information.

The control unit 208 controls all operations of the device 200 for inducing sleep according to the present disclosure. In addition, when a sleep-related signal is obtained through the sensing unit 204, the control unit 208 may transmit the obtained sleep-related signal to the external device 100 through the communication unit 201 and receive sleep state information of the user according to analysis of the sleep-related signal from the external device 100. In addition, the control unit 208 may generate a control signal that changes an output ratio of the first sound source to the second sound source both constituting the synthesized sound source based on the received sleep state information of the user and transmit the control signal to the output unit 205.

The power supply unit 209 may supply power to the device 200 for inducing sleep and include a rechargeable battery module.

The external device 100 may communicate with the device 200 for inducing sleep, and include a communication unit 121, a sensing unit 124, a display unit 125, and a data processing unit 128. The external device 100 may be any computing device capable of performing wired and/or wireless communication. For example, the external device 100 may include a smartphone, a mobile phone, a laptop computer, a personal digital assistant (PDA), a portable multimedia player (PMP), a tablet PC, other wearable devices, etc. Additionally, the external device 100 may include a preset application to interact with the device 200 for inducing sleep or may be connected to a server to download a preset application.

The sensing unit 124 of the external device 100 may include, for example, a temperature sensor, a humidity sensor, an illumination sensor, a noise detection sensor, etc. to obtain information related to an environment of a sleep place. The data processing unit 128 of the external device 100 may analyze the sleep-related signal obtained from the device 200 for inducing sleep, determine a sleep state of the user based on a result of the analysis, and transmit information about the sleep state to the device 200 for inducing sleep. The display unit 125 of the external device 100 may visually display screen information indicating the sleep state, and display history information of a sleep pattern in response to an inquiry request by the user.

In one embodiment, in response to detecting that the device 200 for inducing sleep is worn on a part of the user's body, the device 200 for inducing sleep may be connected to the external device 100 to perform communication. To do so, the device 200 for inducing sleep may be pre-registered for the external device 100 or the external device 100 may be pre-registered for the device 200 for inducing sleep. Alternatively, the device 200 for inducing sleep may be connected to the external device 100 by executing a preset application installed on the external device 100. In addition, in one embodiment, in response to detecting that the device 200 for inducing sleep is separated from the user's body, the device 200 for inducing sleep may be disconnected from the external device 100.

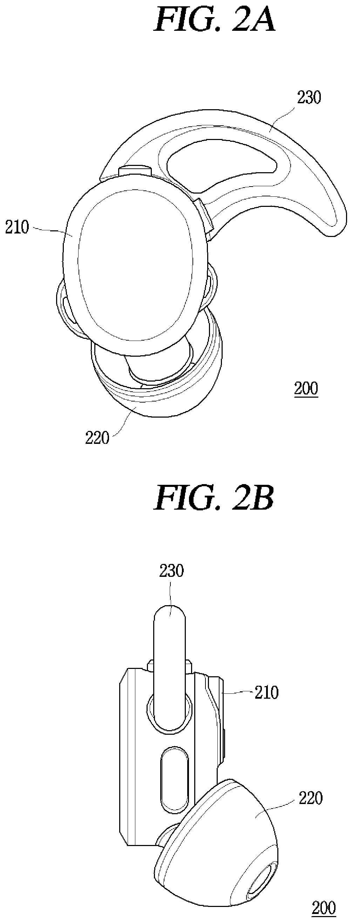

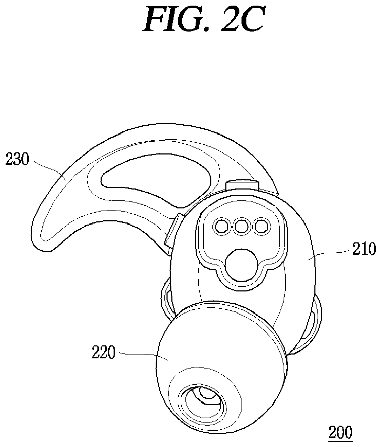

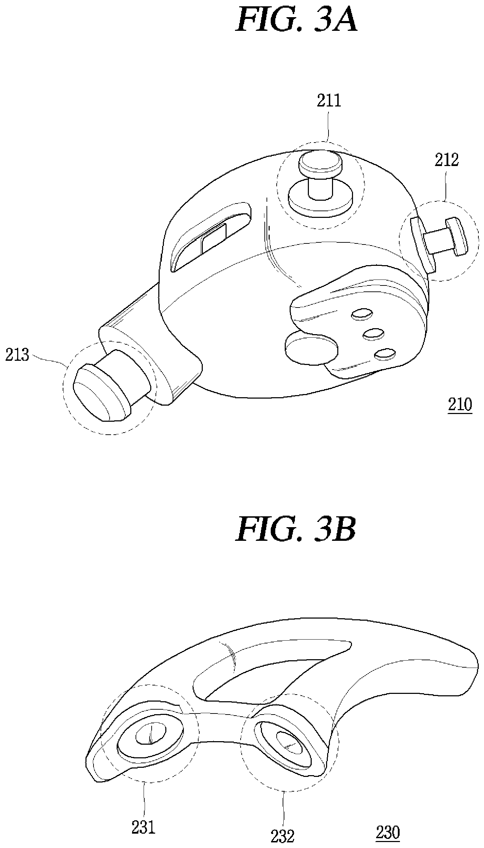

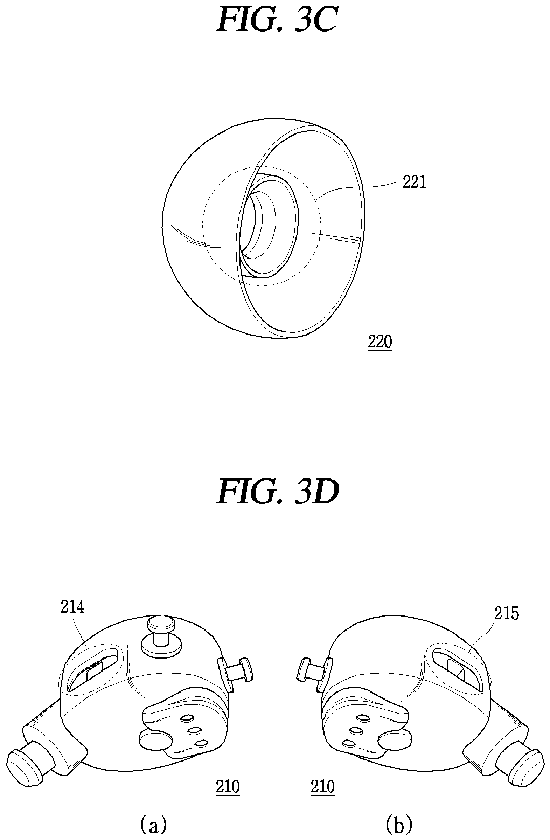

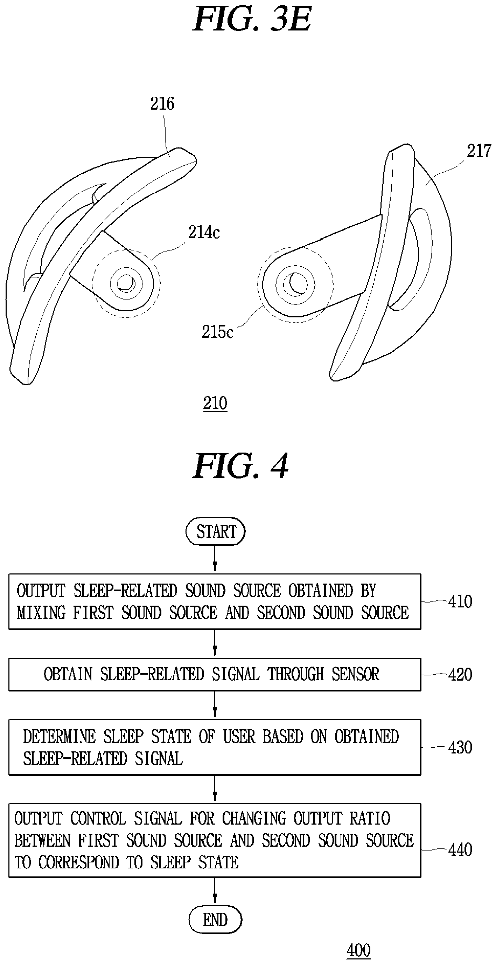

FIGS. 2A, 2B, and 2C illustrate the device for inducing sleep according to the present disclosure viewed from different directions. FIGS. 3A, 3B, 3C, 3D, and 3E are drawings for explaining a detailed configuration of at least some elements disassembled from the device for inducing sleep according to the present disclosure.

As shown in FIGS. 2A to 2C, the device 200 for inducing sleep according to the present disclosure is implemented in a form of an earbud that may be worn on ears of the user. The device 200 for inducing sleep may have a wiring structure divided into a first module 210, a second module 220, and a third module 230 with reference to electrode placement and a contact point when being worn.

In detail, the first module 210 may constitute a main body of the device 200 for inducing sleep and include an EEG sensor configured to measure an EEG signal which is one of sleep-related signals, a conductor, and/or a bias electrode. The second module 220 may include the measurement electrode (SIG) configured to measure a biomedical signal (e.g., an EEG signal), and be configured to be in contact with an inner ear when being worn. The third module 230 may include a reference electrode (REF) sensor and be configured to in contact with back of an ear when being worn. The first to third modules 210, 220, and 230 may have a wiring structure to be decoupled or coupled. In one embodiment, the second module 220 and the third module 230 may be disassembled from the first module 210, i.e., the main body, and then, cleaned and replaced. When the device 200 for inducing sleep is worn on the user's ear, the second module 220 is in contact with the inner ear of the user and the third module 230 is in contact with the back of the ear to provide support to maintain the wearing of the device 200 for inducing sleep.

As illustrated in FIGS. 3A to 3C, the first module 210 constituting the main body of the device 200 for inducing sleep may include a first fastening portion 213 coupled to the second module 220, and second fastening portions 211 and 212 coupled to the third module 230. Here, a frame of the first module 210 may be made of a silicone material (e.g., ultra soft silicone) to provide wearing comfort, and the first and second fastening portions 211, 212, and 213 may be made of a conductor, e.g., a metal material. In addition, although not illustrated, the first module 210 may include the output unit, the power supply unit, the control unit, the communication unit, the signal processing unit, and the memory unit each described with reference to FIG. 1.

As illustrated in FIG. 3C, the second module 220 includes a measurement area 221 in contact with inside of the user's ear in a state of being coupled to the first module 210. The measurement area 221 includes the measurement electrode (SIG) configured to measure a biomedical signal (e.g., an EEG signal). The second module 220 is illustrated as being fit into the first fastening portion 213 of the first module 210 but is not limited thereto. Additionally, to accurately measure brain waves, the second module 220 may include a plurality of measurement electrodes (SIG) (e.g., four measurement electrodes arranged to be apart from each other). In one embodiment, the second module 220 may be manufactured to have various sizes depending on a size of the user's ear and disassembled from the first module 210 to be cleaned.

As illustrated in FIG. 3B, the third module 230 may be configured such that the first portion 231 and the second portion 232 of the third module 230 are fit into the second fastening portions 211 and 212 of the first module 210, respectively. The third module 230 may be made of a conductive silicon material (e.g., a conductive silicon material obtained by mixing a metal powder including carbon and graphite and having a resistance value of 1 Ω or less), and the reference electrode (REF) sensor may be disposed in the first portion 231 and the second portion 232 of the third module 230.

FIGS. 3D and 3E illustrate the first module 210, i.e., the main body of the device 200 for inducing sleep in detail. For convenience of description, the second module 220 and the third module 230 both described above are removed (or disassembled). The device 200 for inducing sleep according to the present disclosure may include the first module 210 implemented in a form of a pair to correspond to a left ear and a right ear of the user as illustrated in (a) and (b) of FIG. 3D. The first module 210 may include third fastening portions 214 and 215 capable of coupling silicon electrode modules 216 and 217 illustrated in FIG. 3E to a side surface. The silicon electrode modules 216 and 217 may include measurement electrode portions 214c and 215c including a plurality of bias electrodes (e.g., four electrodes spaced apart from each other to perform accurate measurement) configured to measure a sleep-related signal. The silicon electrode modules 216 and 217 are coupled such that the measurement electrode portions 214c and 215c are bonded to the third fastening portions 214 and 215 respectively corresponding thereto to be fastened to a printed circuit board (PCB) of the first module 210 using a screw, etc.

Hereinafter, a method of operating the device for inducing sleep having the structure described above to induce sleep is to be described. FIG. 4 is a flowchart for explaining a method 400 of operating the device for inducing sleep according to the present disclosure.

The device for inducing sleep according to the present disclosure communicates with one or more external devices to output a sleep-related sound source ('a synthesized sound source') obtained by mixing a first sound source for inducing sleep with a second sound source other than the first sound source through an output module (e.g., a speaker) (410). To do so, the device for inducing sleep according to the present disclosure may communicate with one or more external devices. For example, the one or more external devices may interoperate with the device for inducing sleep using a preset application installed on the one or more external devices. In this case, the first sound source and the second sound source may be presented to the one or more external devices through a user interface provided by the preset application and selected through a user input.

Then, a sleep-related signal is obtained through a sensor of the device for inducing sleep (420). In one embodiment, the obtaining of the sleep-related signal may be continuously performed while wearing of the device for inducing sleep is detected. In detail, the sensor of the device for inducing sleep may be activated when the device for inducing sleep is worn, and deactivated when the device for inducing sleep is detached.

The sensor of the device for inducing sleep may include one or more sensors configured to obtain a signal corresponding to movement of a user, for example, a gyro sensor, an acceleration sensor, and one or more sensors configured to obtain a biomedical signal of the user, e.g., an EEG, electroconductance (EEC), PPG, ECG, or galvanic skin reflex (GSR) sensor, etc.

An electroencephalogram (EEG) signal is an electrical signal generated on a skin surface by a brain activity according to concentration or external stimulation. An electroencephalography signal may be measured by inducing, on a scalp, a potential fluctuation that occurs in a person's cerebrum or a brain current generated by the potential fluctuation. EEG may be classified into six types according to frequency characteristics. In general, a delta type indicates 'a sleep state', a theta type indicates 'a sleepy state', an alpha type indicates 'a relaxed state', a low beta type indicates 'a concentrated state', a medium beta type indicates 'an alert state', and a high beta type indicates 'an excited state.' That is, a sleep state of an individual user may be estimated through the EEG.

An electrocardiogram (ECG) signal is an electrical signal generated on a skin surface by an electrical activity of a heart. The electrocardiogram signal may be measured by guiding an action current generated from a myocardium according to a heartbeat to two appropriate locations on a body surface. When change characteristics of a period and a waveform of EGC is periodically observed, a psychological state of a user wearing a wearable device, etc. may be distinguished. Additionally, an electromyogram (EMG) signal is an electrical signal generated by muscle contraction force, a muscle activity, and fatigue on a skin surface. Electromyography may detect movement of tendons according to movement of fingers of the user, the movement being detected by wearing a wearable device, etc. In this case, gestures made by the fingers may be determined based on detected information. Additionally, a galvanic skin reflex (GSR) signal is an electrical signal generated on a skin surface by a change in skin resistance according to activation of a sympathetic nerve system. A skin conductance signal may be obtained by measuring a phenomenon such as a temporary decrease in generated electrical resistance or occurrence of an action potential each caused by external stimulation or emotional excitement in skin of a living body. When the user is nervous/enters wakefulness, and thus, a sympathetic nervous system is activated, sweat glands on a skin surface are activated to increase conductivity, and thus, the GSR increases. Additionally, heart rate variability (HRV) is an electrical signal generated on a skin surface by a change in an interval (R-R interval (RRI)) between R-peaks of electrocardiogram. A frequency domain power spectrum of the heart rate variability may be obtained by Fourier transforming a time series signal of the RRI. A low frequency (LF: 0 to 0.15 Hz) domain of this frequency domain power spectrum mainly reflects an activity of the sympathetic nervous system, and a high frequency (HF: (0.15 to 0.4 Hz) domain indicates an activity of a parasympathetic nervous system. In addition, a pulse wave (photoplethysmogram (PPG)) signal is an electrical signal obtained by measuring repeated increases and decreases in arterial blood volume in a fingertip blood vessel in synchronization with a heartbeat. Transmitted light detected at a light receiving portion of a tip of a finger is received after being subtracted in correspondence with an amount of light absorbed by the finger, and presented as a wave form of a blood flow change synchronized with the heartbeat, and this waveform is PPG.

As described above, the sensor configured to obtain a biomedical signal of the user may operate in combination with a plurality of electrodes arranged to be spaced apart from each other on the device 200 for inducing sleep. For example, the EEG sensor is included inside the first module 210 corresponding to the main body of the device 200 for inducing sleep. The plurality of electrodes coupled to the EEG sensor through a conductor and in contact with the user's body are included in the second module 220 or the third module 230. In other words, the EEG sensor and the electrodes are actually disposed in different positions (that is, the EEG sensor is not actually detachably coupled). However, in the present disclosure, since the sensing unit 204 or the sensor includes a plurality of electrodes configured to obtain a biomedical signal, it may be understood that the sensing unit 204 or the sensor is disposed to include a wiring structure to be detachably coupled to the main body.

Based on various biomedical signals detected in this way, the device 200 for inducing sleep according to the present disclosure may continuously check a sleep state and a sleep stage of the user. In addition to the biometric signals described above, a sleep state and a sleep stage of the user may be determined in combination with signals related to movement obtained through a motion sensor, an acceleration sensor, a gyro sensor, etc., and a sleep pattern of the user may be generated.

Then, the device 200 for inducing sleep according to the present disclosure may determine a sleep state of the user based on the obtained sleep-related signal through the external device 100 in interoperation therewith and receive information about the determined sleep state (430). As described above, the sleep state is largely divided into a REM sleep state and a non-REM sleep state. The non-REM sleep state may be subdivided into four stages depending on a sleep stage. Since different brain wave signals appear depending on a sleep stage of the user, the sleep state and sleep stage may be continuously monitored based on the sleep-related signal obtained, for example, by the EEG sensor.

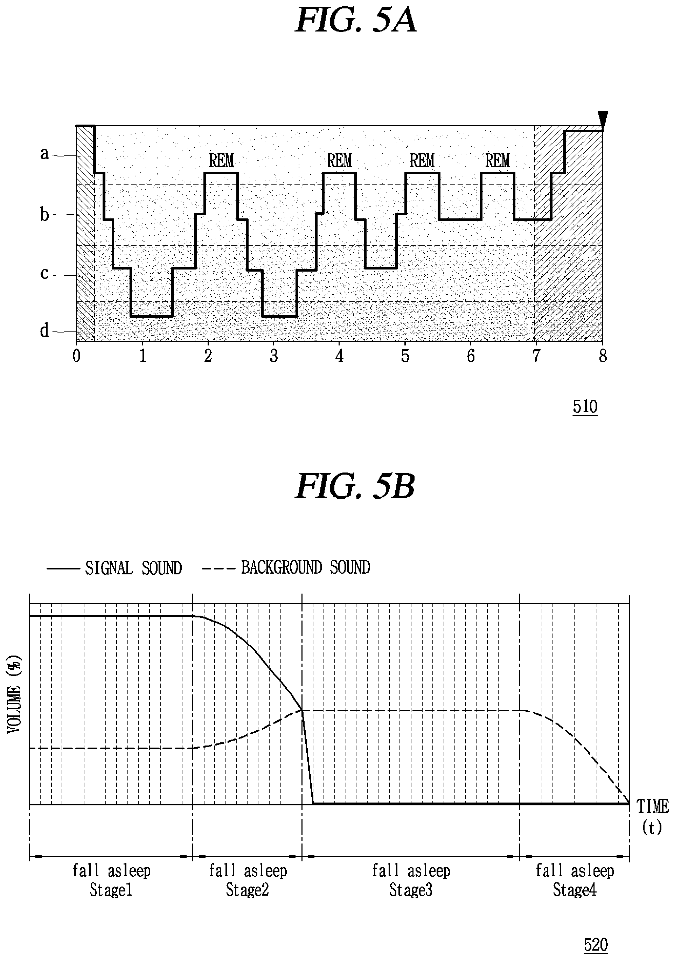

Then, the device 200 for inducing sleep according to the present disclosure outputs a control signal for adjusting an output ratio between the first sound source and the second sound source to correspond to information about the sleep state (440) to thereby control to output a changed synthesized sound source. In relation to this, a method of adjusting a synthesized sound source according to the present disclosure is described in detail with reference to FIGS. 5A and 5B.

FIG. 5A is a sleep graph 510 showing an example of a sleep state and a sleep stage each corresponding to a biomedical signal of a user detected through the device for inducing sleep according to the present disclosure. FIG. 5B is an output graph 520 indicating a synthesized sound source and showing an example of adjusting the synthesized sound source according to the sleep state and the sleep stage of the user.

As shown in FIG. 5A, a sleep state is divided into a REM sleep state and a non-REM sleep state based on a brain wave pattern observed during a sleep activity of the user. The non-REM sleep state is generally subdivided into four stages a, b, c, and d according to a depth of sleep. When sleep initiation is detected in a first step a, beta waves being observed decreases, and alpha waves being observed increases. Additionally, as a sleep stage increases from the first stage a to a fourth stage d, emission of delta waves increases to enter a deep sleep state. Additionally, even after entering the stages c and d for deep sleep, the sleep state may be repeatedly changed to the stages a and b for light sleep on a non-periodical basis.

### [Sleep onset stage]

In the sleep onset stage, the device 200 for inducing sleep according to the present disclosure provides, as sleep-inducing sound, a synthesized sound source obtained by mixing with a second sound source, i.e., a customized sound source that is familiar and comfortable to the user, instead of using only a first sound source for inducing sleep. Accordingly, since the user may start sleeping while listening to a sound source suitable to the user's taste, the user may psychologically comfortably initiate sleep. This may provide an effect of further shortening sleep initiation time. In an embodiment, the device 200 for inducing sleep determines a type and an initial volume value of the first sound source based on a type and an initial volume value of the second sound source selected through a user input and synthesizes the first sound source and the second sound source to provide a result of the synthesizing.

### [Sleep initiation stage and post-sleep initiation stage]

After detecting the sleep initiation, the device 200 for inducing sleep according to the present disclosure operates to adjust an output ratio between the first and second sound sources to induce deep sleep when a light sleep stage (e.g., the stage a or b) is observed based on a sleep-related signal, and operates to turn off the second sound source and increase output of the first sound source to maintain a deep sleep stage when the sleep stage is analyzed as the deep sleep stage (e.g., the stage c or d). Additionally, when the REM sleep is detected, an operation may be performed to output a third sound source to help improvement of memory until non-REM sleep is detected.

In one embodiment, when it is determined that the user has entered a sleep initiation state based on sleep state information of the user, the device 200 for inducing sleep may increase an output ratio of the first sound source to the second sound source at a preset rate to output a synthesized sound source. For example, volume of the first sound source corresponding to a background sound source for inducing sleep, for example, binaural beats, white noise, or rain sound ASMR may be gradually increased. This may be checked in a region of FIG. 5B in which the sleep stage transitions from a stage 1 to a stage 2.

As such, while the volume of the first sound source is gradually increased, the device 200 for inducing sleep according to the present disclosure may proportionally reduce volume of the second sound source corresponding to the user's preferred sound and mixed with the first sound source. In this case, an increase rate of the volume of the first sound source may be different from a decrease rate of the volume of the second sound source. The increase rate of the volume of the first sound source and the decrease rate of the volume of the second sound source may be determined differently depending on a change rate of a sleep stage identified based on a sleep-related signal obtained by the device 200 for inducing sleep.

In one embodiment, the device 200 for inducing sleep according to the present disclosure may control the output module so that the volume of the second sound source is gradually decreased to be turned off while the output ratio of the first sound source to the second sound source is gradually increased. For example, as a result of monitoring a sleep-related signal, when it is estimated that the user is to quickly enter a deep sleep stage, the device 200 for inducing sleep may operate to reduce the volume of the second sound source more quickly than that of the first sound source or quickly turn off the second sound source. On the other hand, as a result of monitoring a sleep-related signals, when the user cannot enter the deep sleep stage or it is estimated that more time is to be consumed, the device 200 for inducing sleep may operate to reduce the volume of the second sound source at a same speed or a lower speed compared to a speed of decreasing the first sound source.

In one embodiment, the device 200 for inducing sleep according to the present disclosure may continuously communicate with the external device to monitor a sleep state corresponding to a sleep-related signal obtained after entry into the sleep initiation state, and differently adjust an increase rate of an output ratio of the first sound source based on updated sleep state information. To do so, the device 200 for inducing sleep may calculate a change rate of the obtained sleep-related signal (or calculate a change rate by the connected external device 100) and change the increase rate with respect to the first sound source in correspondence with a speed of entry from a light sleep stage into a deep sleep stage. For example, in a case of quick entry from the light sleep stage into the deep sleep stage, an output may be controlled to reduce a size of a region of a sleep stage 3 (or the stages 2 and 3) shown in the output graph 520 of FIG. 5B. In a case of a quick entry from the deep sleep stage into the light sleep stage, an output may be controlled to increase a size of a region of the sleep stage 3 (or the stages 2 and 3).

As illustrated in FIG. 5A, a user wearing the device 200 for inducing sleep according to the present disclosure repeats the light sleep stage, the deep sleep stage, and REM sleep during a sleep activity. Accordingly, the output graph 520 indicating the synthesized sound source, illustrated in FIG. 5B, shows only an example in which a sleep stage of the user gradually changes to a deep sleep stage, but the present disclosure is not limited thereto. As shown in the sleep graph 510 of FIG. 5, at a point where a sleep stage is changed back from 'the deep sleep stage' to 'the light sleep stage' (e.g., 1 -> 2 and 3 -> 4 on an x-axis of the sleep graph 510), the output ratio (or volume) of the first sound source may be readjusted based on a sleep state corresponding to a sleep-related signal. Therefore, as the deep sleep stage is observed through the device 200 for inducing sleep according to the present disclosure, the first sound source whose output was increased may be decreased back (a sleep stage 4 in the output graph 520 of FIG. 5B), and then, in response to observing the light sleep stage again, an output of the first sound source may be gradually increased again to induce the deep sleep stage. In this case, an output off of the second sound source may be maintained, or the second sound source may be replaced by a third sound source to improve memory.

In one embodiment, in response to the user's sleep stage reaching a preset level or above based on sleep state information of the user, the device 200 for inducing sleep according to the present disclosure may output a synthesized sound source such that output of the second sound source is turned off and output of the first sound source is gradually reduced to be turned off. This is an operation corresponding to the sleep stage 4 of the output graph 520 of FIG. 5B. However, as described above, when a change in a sleep state is detected by the device 200 for inducing sleep according to the present disclosure, the output of the first sound source may be gradually increased back and the first sound source may be maintained in an off state or replaced by a third sound source.

In one embodiment, while a REM sleep state of the user is monitored based on the sleep state information of the user, the device 200 for inducing sleep according to the present disclosure may output a sound source (a 'third sound source') for inducing improvement of a memory, instead of the synthesized sound source, and when the REM sleep state is complete, operate to provide a synthesized sound source adjusted based on the sleep state information of the user. In this case, in the adjusted synthesized sound source, a second sound source may remain in an off state, and only an output ratio of the first sound source may be adjusted. Alternatively, while the output ratio of the first sound source is gradually increased, the first sound source may be output at a fine output ratio or replaced by a third sound source to be output. As such, adjustment of a sound source constituting a synthesized sound source and volume of the sound source may be updated in a form of various combinations according to a change in a sleep stage.

### [Wake-up preparation stage]

Meanwhile, the device 200 for inducing sleep according to the present disclosure not only induces comfortable and deep sleep of the user, but also operates to induce a seamless and comfortable wake-up.

In detail, the device 200 for inducing sleep according to the present disclosure may maintain an output module configured to output a synthesized sound source to be in an inactivated state, in response to a sleep stage of the user reaching a preset level or above (e.g., the sleep stage 2 or 3 in the sleep graph 510 of FIG. 5A) based on the sleep state information of the user. In addition, when information about an alarm time is received from the external device 100 connected thereto, the device 200 for inducing sleep according to the present disclosure may determine a time point when the output module is to be switched to an activated state based on the received information about the alarm time. The device 200 for inducing sleep according to the present disclosure may operate to switch the output module to the activated state at the time point determined based on the received information about the alarm time and output a fourth sound source for inducing wake-up of the user through the output module switched to the activated state.

In the wake-up preparation stage, the device 200 for inducing sleep according to the present disclosure may perform an operation for inducing wake-up based on remaining sleep time calculated based on total sleep time after sleep initiation and/or a set alarm time. In this case, even when a sleep stage observed through the sleep induction device 200 is same, different operations may be performed depending on the total sleep time and/or the set alarm time after the sleep initiation.

For example, when the total sleep time after the sleep initiation reaches a usual sleep pattern, (even when an alarm is not set,) an operation may be performed to output a sound source or a brainwave stimulation signal for inducing wakefulness even in a deep sleep stage. In addition, for example, when remaining sleep time calculated based on notification time reaches a preset value (e.g., 15 minutes before), an operation may be performed to output a fourth sound source for inducing wake-up. In this case, the fourth sound source is a sound source that changes a brainwave signal corresponding to a current sleep stage and may be determined differently depending on the current sleep stage. For example, in the wake-up preparation stage, when a sleep stage observed through the device 200 for inducing sleep is a 'deep sleep stage' (when 'delta waves' are dominant), a sound source for inducing 'alpha waves' (or 'theta waves') for inducing a light sleep stage is output, and then, the sound source is gradually changed to a sound source for inducing 'beta waves' to be output so that the user may wake up naturally when the alarm time is reached. Also, for example, in the wake-up preparation stage, when a sleep stage observed through the device 200 for inducing sleep is a light sleep stage or corresponds to REM sleep, an operation may be performed to immediately output a sound source for inducing 'beta waves.'

Meanwhile, the device 200 for inducing sleep according to the present disclosure may operate to communicate with one or more external devices 100 and operate in cooperation with a preset application executed through the one or more external devices 100.

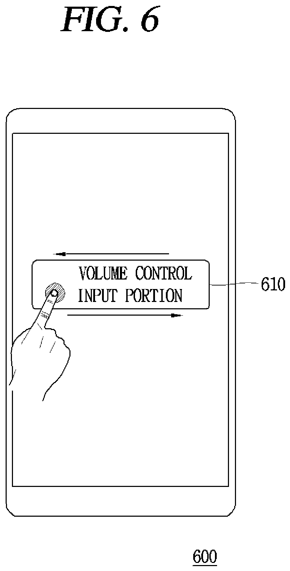

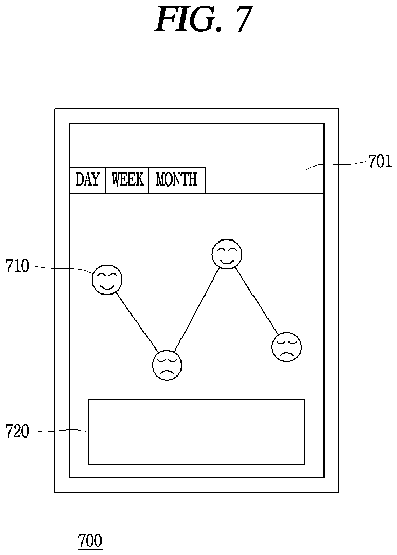

FIG. 6 illustrates an example of a user interface related to a method 600 of controlling a synthesized sound source in an external device that communicates with the device for inducing sleep according to the present disclosure. FIG. 7 illustrates an example of a user sleep analysis screen 701 related to a method 700 of displaying a sleep pattern in an external device.

The device 200 for inducing sleep according to the present disclosure operates to transmit information related to a user interface for determining an initial volume value of a selected second sound source to the external device 100 connected to the device for inducing sleep. The device for inducing sleep determines an output ratio of a first sound source based on a type of the selected second sound source and an initial volume value of the second sound source determined using the user interface and operates to output a synthesized sound source obtained accordingly.

In detail, the device 200 for inducing sleep communicates with the external device 100 to transmit a sleep-related signal obtained from the device 200 for inducing sleep. The external device 100 analyzes the received sleep-related signal and transmits sleep state status information. As described above, the device 200 for inducing sleep according to the present disclosure may operate in cooperation with a preset application executed on the external device 100, and the first sound source and the second sound source may be prestored in the memory unit of the device 200 for inducing sleep or transmitted from the external device 100 at request of the device 200 for inducing sleep. The device 200 for inducing sleep may generate a synthesized sound source by mixing the first sound source with the second sound source and transmit the generated synthesized sound source and information related thereto to the external device 100 to output an initial output ratio of the synthesized sound source to an application programming interface (API) for being executed by one click/input.

Referring to FIG. 6, an output ratio adjustment button 610 for a synthesized sound source is provided on the external device 100 being connected. An image requiring a left/right drag input (a sliding input) in a form of a movement element/arrow may be output on the output ratio adjustment button 610. When a drag input is applied onto the output ratio control button 610 in a left direction, volume of the second sound source, i.e., preferred sound of the user is decreased, and an output ratio of the first sound source is decreased together in proportion to the decrease of the second sound source to provide a uniform synthesized sound source. On the other hand, when a drag input is applied onto the output ratio control button 610 in a right direction, volume of the second sound source, i.e., preferred sound of the user is increased, and an output ratio of the first sound source is increased together in proportion to the increase of the second sound source to also provide a uniform synthesized sound source. A value of an initial output ratio corresponding to such an input is transmitted to the device 200 for inducing sleep through the external device 100, and the device 200 for inducing sleep outputs a synthesized sound source at a set initial output ratio. As such, the user may set and execute the initial output ratio of the synthesized sound source with one intuitive input without having to select each sound source of the synthesized sound source for inducing sleep or adjust volume of the synthesized sound source.

Referring to FIG. 7, the device 200 for inducing sleep according to the present disclosure provides a sleep-related signal and information about an output of a synthesized sound source corresponding to a sleep state to the connected external device 100 to receive a sleep pattern analysis result. In FIG. 7, the sleep pattern analysis screen 701 may be checked for each period (day, week, month), and in the sleep pattern analysis screen 701, for example, a graph image 710 representing 'a light sleep stage' and 'a deep sleep stage' during sleep activity time of the user and sleep analysis information 720 in a text form may be provided. The sleep pattern analysis screen 701 is provided to improve the user's sleep. To do so, in one embodiment, the device 200 for inducing sleep may collect information regarding a degree of adjustment of an output ratio between the first and second sound sources during a preset period, output a synthesized sound source updated based on the collected information, and transmit relevant information to the external device 100 to perform continuous sleep improvement learning.

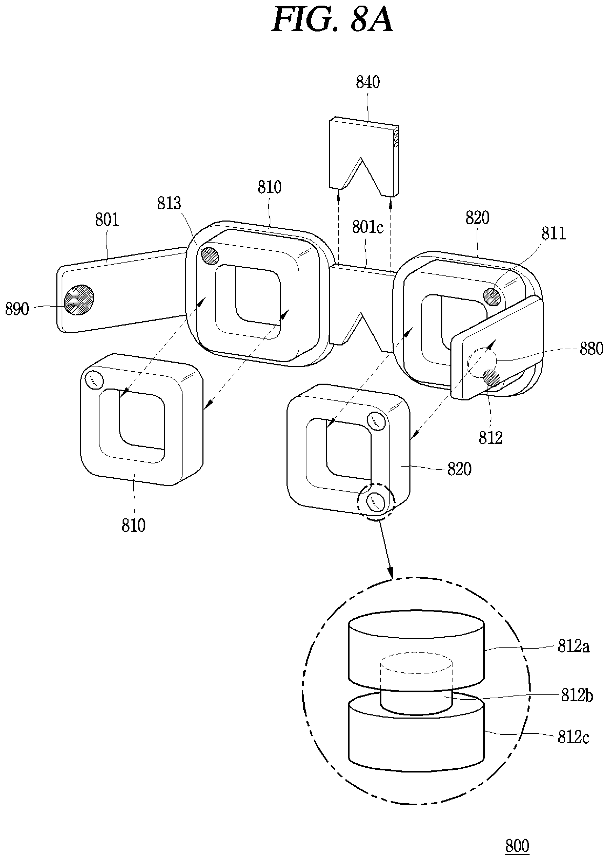

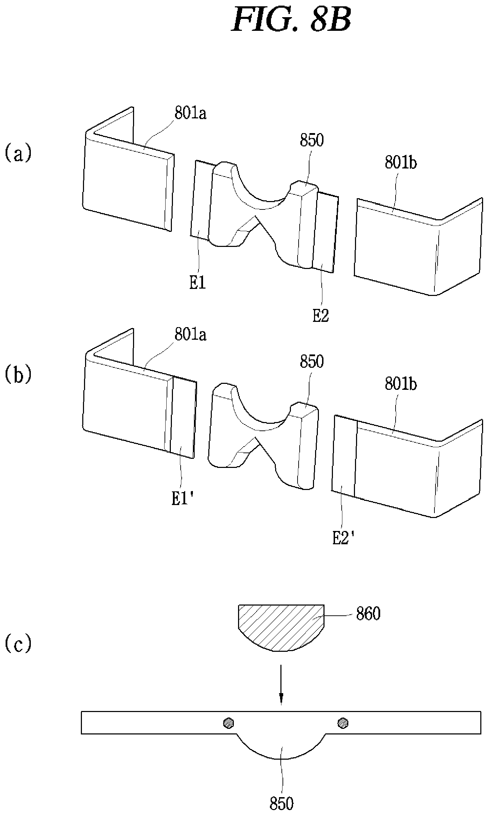

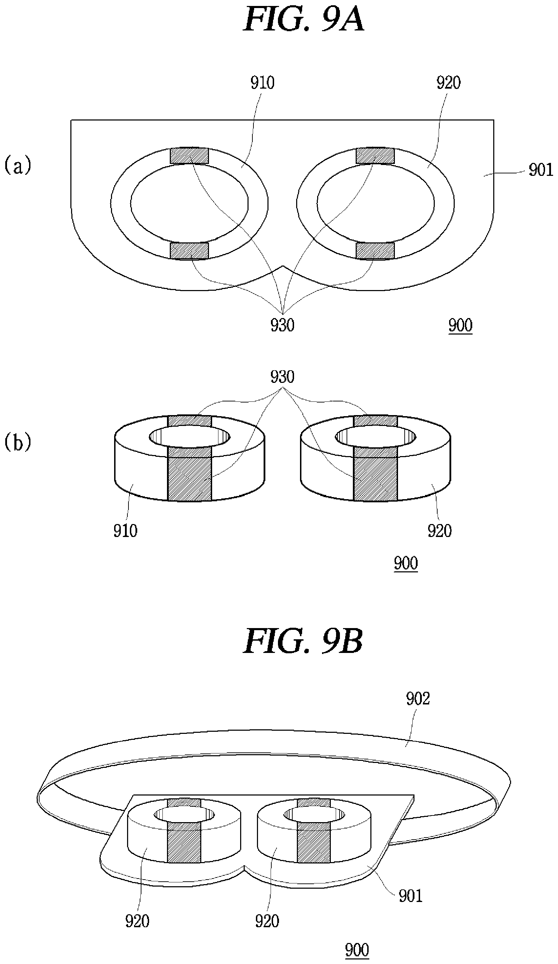

Meanwhile, the device for inducing sleep according to the present disclosure may be implemented not only in the form of the earbud described above, but also alternatively/additionally in the form of glasses or eye patches. FIGS. 8A and 8B illustrate a glasses-type device for inducing sleep. FIGS. 9A and 9B illustrate an eye patch-type device for inducing sleep. In the form of the ear bud described above, 'an inner side and a rear side of an ear' are used as a point of contact with a body of a user for measuring an EEG signal. However, in the glasses-type or eye patch-type device for inducing sleep to be described later, 'a periphery of eyes' may be selected as a point of contact with a body of a user for measuring an EEG signal.

As illustrated in FIGS. 8A and 8B, a glasses-type device 800 for inducing sleep may include a main body 801, a first region 810 and a second region 820 in correspondence with both eyes of the wearer to measure a sleep-related signal and including an electrode, respectively, a connection portion 801c, and first and second speakers 880 and 890 in correspondence with both ears of the wearer to output a synthesized sound source. In addition, although not illustrated, a control circuit unit, a display module, an input module, a battery module, etc. may be further included.

The first region 810 and the second region 820 are configured to be detachably coupled to the main body 801 and may be made of an elastic material capable of minimizing wearing discomfort and providing uniform pressure while in contact with an electrode, e.g., a sponge, memory foam, or a silicone material. In addition, a plurality of electrodes 811, 812, and 813 are configured to obtain an EEG signal in contact with the periphery of the eyes of the user and respectively disposed in the first region 810 and the second region 820. As described above, dry electrodes capable of being cleaned are used. A plurality of measurement electrodes (SIG) may be disposed in an area corresponding to a left (right) eye and a reference electrode (REF) and a ground electrode (GND) may be disposed in an area corresponding to a right (left) eye to be spaced apart from the plurality of measurement electrodes (SIG). Additionally, the plurality of measurement electrodes (SIG_ may be disposed to be spaced apart from each other to increase data reliability.

In one embodiment, each of the plurality of electrodes 811, 812, and 813 may be divided into an electrode portion 812a detachably coupled to the main body 801 and capable of being cleaned, a contact point portion 812b corresponding to a contact point of an electrode, and a support portion 812c made of an elastic material to include a mesh air/spring or a structure such as a springboard to provide elastic force and uniform pressure. Additionally, the glasses-type device 800 for inducing sleep may include an expansion module connection portion 840 detachably coupled to the connection portion 801c connecting the first region 810 to the second region 820. As shown in (c) of FIG. 8B, the expansion module connection portion 840 may be configured such that an expansion module 860 such as an EEG addition channel, a module configured to measure photoplethysmography (PPG), a body temperature sensor, an electrical stimulation sensor, etc. may be coupled into a fit part 850 of the expansion module connection portion 840.

Additionally, in one embodiment, the eyeglass-type device 800 for inducing sleep may be implemented to have a structure that allows size adjustment in consideration of a distance between eyes of the wearer, a size of a face/head, etc. To do so, as illustrated in (a) of FIG. 8B, first and second expansion connection portions E1 and E2 may be included on left and right sides of the fit part 850 of the connection portion 801c seated on a nose of the wearer and connecting the first and second regions 810 and 820 with each other. Alternatively, as shown in (b) of FIG. 8B, each of the first and second regions 810 and 820 may include an expansion connection portion in a position fit into the fit part 850. That is, a first extension connection second E1' disposed in the connection portion 801a of the first region 810 and a first extension connection portion E2' disposed in the connection portion 801b of the second region 820 may be included.

As illustrated in FIGS. 9A and 9B, an eye patch-type device 900 for inducing sleep includes a first region module 910 and a second region module 920 detachably coupled to a main body 901 of an eye patch-type and disposed to correspond to both eyes of a wearer and has a wiring structure. In each of the first region module 910 and the second region module 920, a plurality of electrodes configured to obtain sleep-related signals are disposed to be apart from the contact portion 930 configured to be connected to the main body 901. In addition, a speaker (e.g., a micro-electromechanical systems (MEMS) speaker), a flexible PCB (FPCB), a battery, etc. are disposed in the connection portion 902 of the main body 901. In addition, the first region module 910 and the second region module 920 may be made of a conductive silicone material that may be cleaned.

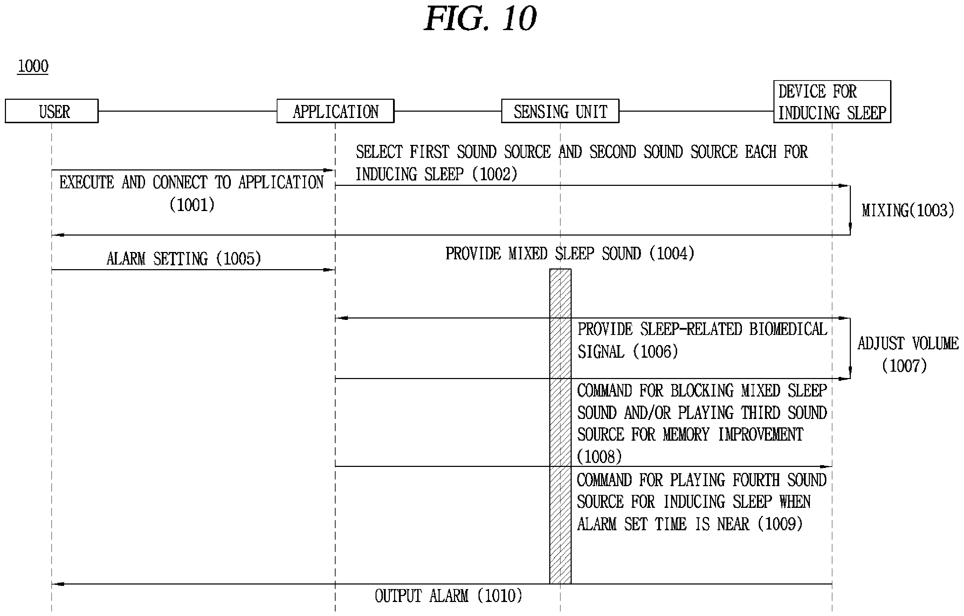

FIG. 10 is a flowchart for explaining a service providing method 1000 for inducing, by the device for inducing sleep according to the present disclosure, sleep and wake-up in interoperation with a preset application.

The service providing method 1000 of FIG. 10 starts with executing an application (e.g., a sleep-inducing application) preset by a user and connecting to the executed application (1001). Then, the device for inducing sleep according to the present disclosure receives a selection of first and second sound sources for inducing sleep through the connected application (1002), mixes the first sound source with the second sound source (1003), and provides a synthesized sound source obtained by the mixing, i.e., a mixed synthesized sound source to a user through an output module (e.g., a speaker) of the device for inducing sleep (1004). Then, information regarding movement of the user and biomedical signals are continuously collected through a sensing unit of the device for inducing sleep. The collected biomedical signals are signal-processed, and then, provided to and stored in the preset application and the device for inducing sleep (1006). An output ratio (or a mixing ratio) between the first sound source and the second sound source both constituting that the synthesized sound source is adjusted according to a sleep state of the user determined based on the collected biomedical signals, and as an example, volume of the synthesized sound source may be adjusted (1007). In detail, volume of the second sound source corresponding to a preferred sound source selected by the user may be reduced, or volume of the first sound source, i.e., a sleep-inducing background sound source that matches the second sound source may be slightly increased. In addition, according to the sleep state of the user determined based on the collected biomedical signals, the device for inducing sleep may operate to block (or turn off) an output of the mixed synthesized sound source through the preset application. In addition, according to the sleep state of the user determined based on the collected biomedical signals, the device for inducing sleep may operate, through the preset application, to play a third sound source for improving memory while in the sleep state (1008). In this way, a sleep-inducing service for inducing deep sleep and improving memory may be provided by adjusting an output ratio between the first sound source and the second sound source according to a sleep state, performing turn-off, or properly providing the third sound source.

Meanwhile, the user may set an alarm using the preset application (1005), and although not illustrated, information about set alarm time may be transmitted to the device for inducing sleep. In this case, when an alarm time set through the preset application is near (e.g., 15 minutes before the alarm time), the device for inducing sleep according to the present disclosure may output a fourth sound source for inducing wake-up of the user until before alarm sound that matches the alarm time is output (1009). In this case, volume and output time of the fourth sound source may be adjusted differently depending on the user's sleep state determined based on information about the user's movement and biomedical signals each continuously collected by the sensing unit. By doing so, the device for inducing sleep according to the present disclosure may further provide a wake-up inducing service for inducing refreshing and comfortable wake-up by gradually changing brain waves before the alarm output (1010).

As described above, according to the device for inducing sleep according to the present disclosure and a method of operating the same, implementation may be performed such that an element in contact with a user's body to measure brain waves may be cleaned and detachably coupled to allow hygienic and stable brain wave measurement. In addition, a conductive silicon material may be used in consideration of wearing comfort to allow accurate and stable brain wave measurement even during sleeping for a long period of time and minimize wearing discomfort. In addition, not only a sound source for inducing sleep, but also a synthesized sound source obtained by uniformly mixing preferred sounds familiar to a user with a sound source for inducing sleep may be properly adjusted and provided according to a sleep state and a sleep stage so that the user may initiate sleep comfortably, have sound sleep, and wake up refreshed. In addition, a personalized and customized sleep sound source may be provided, sleep initiation time may be reduced, deep sleep may be induced, and in a case of preparing wake-up, seamless wakefulness may be induced by stimulating brain waves to enable wakefulness step by step.

Further scope of applicability of the present disclosure will become apparent from the following detailed description. It should be understood, however, that the detailed description and specific examples, such as the preferred embodiment of the disclosure, are given by way of illustration only, since various changes and modifications within the spirit and scope of the disclosure will be apparent to those skilled in the art.

The features, structures, effects, etc. described above in the embodiments are included in at least one embodiment of the present disclosure, but are not limited to only one embodiment. Further, the features, structures, effects, etc. described in the embodiments may also be combined or modified with respect to other embodiments by those of ordinary skill in the art to which the embodiments pertain. Therefore, content related to such combinations and modifications should be construed as being included in the scope of the present disclosure.

In addition, the foregoing description has been made with reference to the embodiments, but it is merely illustrative and is not intended to limit the present disclosure. It will be apparent that other changes and applications can be made by those skilled in the art to which the present disclosure belong without departing from substantial features of the embodiments of the present disclosure. For example, each component specifically shown in the embodiments can be modified and implemented. Therefore, differences related to such modifications and applications should be construed as being included in the scope of the present disclosure as defined in the appended claims.

## Claims

1. A device for inducing sleep, the device comprising:
a main body configured to be wearable on a body of a user;
a communication module configured to communicate with an external device;
a sensor detachably coupled to the main body and configured to obtain a sleep-related signal by being in contact with a part of the body of the user;
an output module configured to output a synthesized sound source obtained by mixing a first sound source for inducing sleep of the user with a second sound source other than the first sound source; and
a control unit configured to control the communication module to transmit the obtained sleep-related signal to the external device and receive, from the external device, sleep state information of the user according to analysis of the sleep-related signal,
wherein the control unit controls to output the synthesized sound source by adjusting an output ratio of the first sound source and the second sound source both constituting the synthesized sound source based on the received sleep state information of the user.

2. The device of claim 1, wherein, in response to determining entry into a sleep initiation state based on the sleep state information of the user, the control unit outputs the synthetic sound source by increasing the output ratio of the first sound source to the second sound source to a preset rate.

3. The device of claim 2, wherein, while the output ratio of the first sound source to the second sound source is gradually increased, the control unit controls the output module to gradually decrease volume of the second sound source to be turned off.

4. The device of claim 2, wherein the control unit continuously communicates with the external device to monitor a sleep state corresponding to a sleep-related signal obtained after the entry into the sleep initiation state and adjusts an increase rate of the output ratio of the first sound source differently based on updated sleep state information.

5. The device of claim 1, wherein, when the second sound source is selected through the external device, the control unit transmits information related to a user interface configured to determine an initial volume value of the selected second sound source to the external device.

6. The device of claim 5, wherein the output ratio of the first sound source is determined differently based on a type of the selected second sound source and the initial volume value of the second sound source, the initial volume value being determined using the user interface.

7. The device of claim 1, wherein, in response to a sleep stage of the user reaching a preset level or above based on the sleep state information of the user, the control unit outputs a synthesized sound source such that an output of the second sound source is turned off and an output of the first sound source is gradually reduced for a certain period of time to be turned off.

8. The device of claim 1, wherein, while a rapid eye movement (REM) sleep state of the user is monitored based on the sleep state information of the user, the control unit controls the output module to output a sound source for inducing improvement of a memory, instead of the synthesized sound source, and when the REM sleep state is complete, to provide a synthesized sound source adjusted based on the sleep state information of the user.

9. The device of claim 1, wherein, in response to a sleep stage of the user reaching a preset level or above based on the sleep state information of the user, the control unit maintains the output module in an inactivated state, and when information about an alarm time is received from the external device, determines a time point when the output module is to be switched to an activated state based on the received information about the alarm time.

10. The device of claim 9, wherein the control unit switches the output module to the activated state at the time point determined based on the received information about the alarm time, and the output module outputs a sound source for inducing wake-up of the user.

11. The device of claim 1, wherein the control unit collects information regarding a degree of adjustment of the output ratio of the first sound source to the second sound source during a preset period of time, and performs learning using the external device to output a synthesized sound source updated based on the collected information.

12. The device of claim 1, wherein the sensor is configured to comprise a wiring structure to be detachably coupled to the main body, and comprises a plurality of electrodes disposed to be apart from each other.

13. The device of claim 12, wherein at least one of the plurality of electrodes is disposed to be in contact with an inner ear of the user, and made of a conductive silicone material.

14. A method of operating a device for inducing sleep wherein the device is configured to be wearable on a body of a user and communicate with an external device, the method comprising:
obtaining a sleep-related signal, the obtaining being performed by a sensor configured to be in contact with a part of the body of the user;
outputting a synthesized sound source obtained by mixing a first sound source for inducing sleep of the user with a second sound source other than the first sound source;
transmitting the sleep-related signal obtained by the sensor to the external device, and receiving sleep state information of the user according to analysis of the sleep-related signal received from the external device; and
outputting a synthesized sound source updated by adjusting an output ratio of the first sound source to the second sound source based on the received sleep state information of the user.

15. A computer program stored on a computer-readable recording medium for performing the method of claim 14 on a computer operated by one or more processors.
